# EUROPEAN PATENT APPLICATION

(11) **EP 2 082 680 A1**
(43) Date of publication of application: **29.07.2009**
(21) Application number: 09001047.1
(22) Date of filing: 26.01.2009
(51) Int. Cl.: A61B 1/05

(54) **Capsule endoscope, method of controlling the same, and information manager**

(30) Priority: 28.01.2008 JP 2008016339
(71) Applicant: FUJIFILM Corporation, Minato-ku Tokyo (JP)
(72) Inventor: Nishino, Naoyuki, Ashigarakami-gun Kanagawa (JP)
(74) Representative: Klunker . Schmitt-Nilson . Hirsch

(57) **Abstract**

A capsule endoscope swallowable in a body (10) is provided, and includes an image pickup device (34) for picking up an image (113, 114) in the body. A transmitter (55) externally transmits information of the image. By use of an acceleration sensor (59), a capsule moving speed is detected in the body. A CPU (50) as pixel number changer changes a pixel number (Pmax, Pmid, Plow) of the image, namely resolution of the image according to information (V, L, ΔL, SI) of the capsule moving speed. The CPU sets the pixel number lower according to lowness of the capsule moving speed (V). Also, the CPU sets the pixel number lower according to lowness of a capsule moving distance (L, ΔL), and/or according to highness of similarity degree (SI) between plural consecutive images. Preferably, the CPU sets the pixel number by at least one of pixel binning, pixel thinning and pixel averaging.

## Description

### BACKGROUND OF THE INVENTION

### 1. Field of the Invention

The present invention relates to a capsule endoscope, a method of controlling the same, and an information manager. More particularly, the present invention relates to a capsule endoscope, a method of controlling the same, and an information manager, in which images can be handled with high efficiency in image reading.

### 2. Description Related to the Prior Art

A capsule endoscope has been recently developed for the purpose of medical inspection of a patient's body. The capsule endoscope includes a capsule body of a very small size, and an image pickup device and a light source incorporated in the capsule body. For the inspection, at first a patient orally swallows the capsule endoscope. The light source illuminates a body part in a gastrointestinal tract of his or her human body. The image pickup device creates an image of the body part. Image data are obtained. A receiver wirelessly receives a radio wave of the image data, and writes the image data in an internal flash memory as data storage. During or after the inspection, the image data is retrieved in a workstation or other information manager, for a doctor or physician to read images displayed on a monitor display panel.

A frame rate of the capsule endoscope, or the number of times of imaging per unit time, is 2 fps (frames per second). The total of the time for the imaging to create images is approximately eight (8) hours or more. A total size of the image data stored in the receiver is considerably large. If all the created images are desired for image reading, excessively much time and load will be required. There are various suggestions for reducing such time and load in the course of image reading, for example, U.S.P. Nos. 6,709,387 and 7,022,067 (corresponding to JP-A 2004-521662 and 2006-223892).

When a moving speed of the capsule endoscope is low, or when there is high similarity degree between two successive images obtained by the capsule endoscope, then the capsule endoscope remains in one position continuously. Plural successive images with very slight difference are created. However, U.S.P. Nos. 6,709,387 and 7,022,067 (corresponding to JP-A 2004-521662 and 2006-223892) disclose operation of lowering the frame rate when the moving speed is low, and operation of raising the display rate when there is high the similarity degree between two successive images. Load for image reading can be reduced, as the number of similar images can be reduced, or the number or time required for reading similar images can be reduced.

It is possible according to U.S.P. Nos. 6,709,387 and 7,022,067 (corresponding to JP-A 2004-521662 and 2006-223892) to reduce the load in the image reading. However, the moving speed of the capsule endoscope is considerably high in view of power for use in wirelessly transmitting the image data from the capsule endoscope to the receiver as a great component of the power for use. If the frame rate is set higher, the power for use becomes higher due to the greater number of times of wireless transmission. The power source will be used up quickly. When the power source is consumed even in the presence of the capsule endoscope in a human body, no succeeding images will be obtained.

The image data are transmitted successively after imaging in consideration of the transmission process time required for transmitting the image data, because the transmission process time is considerably longer than the imaging process time. Imaging of a succeeding image is impossible before completion of the transmission of the image data, so that there is an upper limit of the frame rate. Even if the frame rate is set the highest in a possible range according to the present performance of imaging, the body part with a lesion as a region of interest, which must be read and diagnosed with great importance, can be missed very possibly. When the frame rate is set extremely high, the power for use will be very high. If a region of interest is viewed at the same time as drop of the frame rate, the region of interest is missed due to the interruption in imaging.

### SUMMARY OF THE INVENTION

In view of the foregoing problems, an object of the present invention is to provide a capsule endoscope, a method of controlling the same, and an information manager, in which images can be handled with high efficiency in image reading.

In order to achieve the above and other objects and advantages of this invention, a capsule endoscope swallowable in a body is provided, and includes an image pickup device for picking up an image in the body. A transmitter externally transmits information of the image. A state detector detects a capsule endoscope state in the body. A pixel number changer changes a pixel number of the image according to information of the capsule endoscope state.

The information of the capsule endoscope state is at least one of a capsule moving speed in the body, a capsule moving distance in the body, and similarity degree between plural consecutive images. The pixel number changer compares the information of the capsule endoscope state with a predetermined threshold value, and determines the pixel number according to a result of the comparison.

The pixel number changer changes the pixel number stepwise at least between a maximum pixel number of pixels in the image pickup device and a pixel number lower than the maximum pixel number.

The transmitter transmits the image wirelessly. When the pixel number is set equal to a maximum pixel number of pixels in the image pickup device, the image pickup device and the transmitter operate for imaging of the image and wireless transmission without idle time. When the pixel number is set lower than the maximum pixel number, the image pickup device and the transmitter wait during an idle time associated with the imaging of the image and the wireless transmission.

The transmitter transmits the image wirelessly. The image pickup device and the transmitter operate for imaging of the image and wireless transmission without idle time irrespective of how the pixel number is set.

In one preferred embodiment, the transmitter transmits the image wirelessly. When the pixel number is set equal to a maximum pixel number of pixels in the image pickup device, the image pickup device and the transmitter operate for imaging of the image and wireless transmission without idle time. When the pixel number is set lower than the maximum pixel number, comparison of the information of the capsule endoscope state with the threshold value is considered, for determining a selected one of first and second modes, and in the first mode, the image pickup device and the transmitter wait during an idle time associated with the imaging of the image and the wireless transmission, and in the second mode, the image pickup device and the transmitter operate by shortening the idle time for the imaging of the image at the lower pixel number and the wireless transmission.

The pixel number changer changes the pixel number by pixel thinning.

In another preferred embodiment, the information of the capsule endoscope state is a capsule moving distance in the body. The image pickup device creates first images at a regular distance interval according to the capsule moving distance, and creates a second image between the first images. The pixel number changer sets the pixel number for the first images equal to a maximum pixel number of pixels in the image pickup device, and sets the pixel number for the second image lower than the maximum pixel number.

In still another preferred embodiment, the information of the capsule endoscope state is similarity degree between plural consecutive images. Furthermore, a frame rate changer changes a frame rate of the image pickup device according to the similarity degree. The image pickup device picks up first images at the frame rate set by the frame rate changer, and if the frame rate is made lower than a highest frame rate in the frame rate changer, picks up a second image between the first images. The pixel number changer sets the pixel number for the first images equal to a maximum pixel number of pixels in the image pickup device, and sets the pixel number for the second image lower than the maximum pixel number.

The information of the capsule endoscope state further includes at least one of a capsule moving speed in the body and a capsule moving distance in the body. The frame rate changer sets the frame rate for the second image according to the capsule moving speed or the capsule moving distance.

The transmitter transmits the information of the pixel number associated with the image.

The pixel number changer changes the pixel number by at least one of pixel binning, pixel thinning and pixel averaging.

Furthermore, data storage stores images created variously by imaging in the image pickup device. The transmitter externally transmits the images from the data storage after discharge from the body.

The pixel number changer sets the pixel number lower according to lowness of the capsule moving speed.

The pixel number changer sets the pixel number lower according to lowness of the capsule moving distance.

The pixel number changer sets the pixel number lower according to highness of the similarity degree between plural consecutive images.

Also, a capsule endoscope control method for a capsule endoscope swallowable in a body is provided. The capsule endoscope includes an image pickup device, for picking up an image in the body, and a transmitter for externally transmitting information of the image. In the capsule endoscope control method, a capsule endoscope state is detected in the body. A pixel number of the image is changed according to information of the capsule endoscope state.

The information of the capsule endoscope state is at least one of a capsule moving speed in the body, a capsule moving distance in the body, and similarity degree between plural consecutive images.

Also, an information manager for an image from a capsule endoscope swallowable in a body is provided. The capsule endoscope includes an image pickup device for picking up the image in the body. A state detector detects a capsule endoscope state in the body. A pixel number changer changes a pixel number of the image according to information of the capsule endoscope state. A transmitter externally transmits information of the image and the pixel number. The information manager includes a managing unit for managing the image retrieved from the capsule endoscope, to display the image for image reading. A display control unit changes a displayed form of the image according to the information of the pixel number.

Furthermore, there is an input interface operable externally. The display control unit normally causes displaying an image of a specific pixel number, and when the input interface is operated, causes displaying an image of a pixel number different from the specific pixel number.

The specific pixel number is a maximum pixel number of pixels in the image pickup device.

Also, a capsule endoscope swallowable in a body is provided, and includes an image pickup device for picking up an image in the body. A transmitter externally transmits information of the image. A state detector detects a capsule endoscope state in the body. A resolution changer changes resolution of the image according to information of the capsule endoscope state.

Consequently, images can be handled with high efficiency in image reading, because the pixel number of images, which have relatively low importance, can be lowered in the imaging.

### BRIEF DESCRIPTION OF THE DRAWINGS

The above objects and advantages of the present invention will become more apparent from the following detailed description when read in connection with the accompanying drawings, in which:
Fig. 1A is a front elevation, partially broken illustrating a capsule endoscope and a receiver in a capsule endoscope system together with a human body;
Fig. 1B is a perspective illustrating a workstation with the receiver in the capsule endoscope system;
Fig. 2 is a horizontal section illustrating the capsule endoscope;
Fig. 3 is a block diagram schematically illustrating circuit elements in the capsule endoscope;
Fig. 4 is a block diagram schematically illustrating a CCD;
Fig. 5 is an explanatory view illustrating pixels in the course of the pixel binning process;
Fig. 6 is an explanatory view illustrating addition and rearrangement of red pixels;
Fig. 7 is an explanatory view illustrating addition and rearrangement of blue pixels;
Fig. 8 is an explanatory view illustrating addition and rearrangement of green pixels;
Fig. 9 is a block diagram schematically illustrating a receiver;
Fig. 10 is a block diagram schematically illustrating a workstation;
Fig. 11 is a front elevation illustrating an example of a displayed form of an image;
Fig. 12 is a flow chart illustrating operation of the capsule endoscope;
Fig. 13 is a timing chart illustrating sequences of imaging and wireless transmission;
Fig. 14 is a timing chart illustrating one preferred embodiment in which medium or low pixel number imaging is carried out without idle time;
Fig. 15 is a flow chart illustrating operation of the capsule endoscope of the embodiment;
Fig. 16 is a flow chart illustrating another preferred embodiment in which a moving distance is considered to start the low pixel number imaging;
Fig. 17 is a timing chart illustrating a sequence of creating images in the embodiment of Fig. 16;
Fig. 18 is a timing chart illustrating another sequence of creating images in the embodiment of Fig. 16;
Fig. 19 is a block diagram schematically illustrating still another preferred embodiment in which similarity degree SI of images is considered;
Fig. 20 is a flow chart illustrating operation of a capsule endoscope of the embodiment of Fig. 19;
Fig. 21 is a timing chart illustrating a sequence of creating images in the embodiment;
Fig. 22 is a timing chart illustrating another sequence of creating images in the embodiment; and
Fig. 23 is a block diagram schematically illustrating one preferred embodiment in which data storage is incorporated in a capsule endoscope.

### DETAILED DESCRIPTION OF THE PREFERRED EMBODIMENT(S) OF THE PRESENT INVENTION

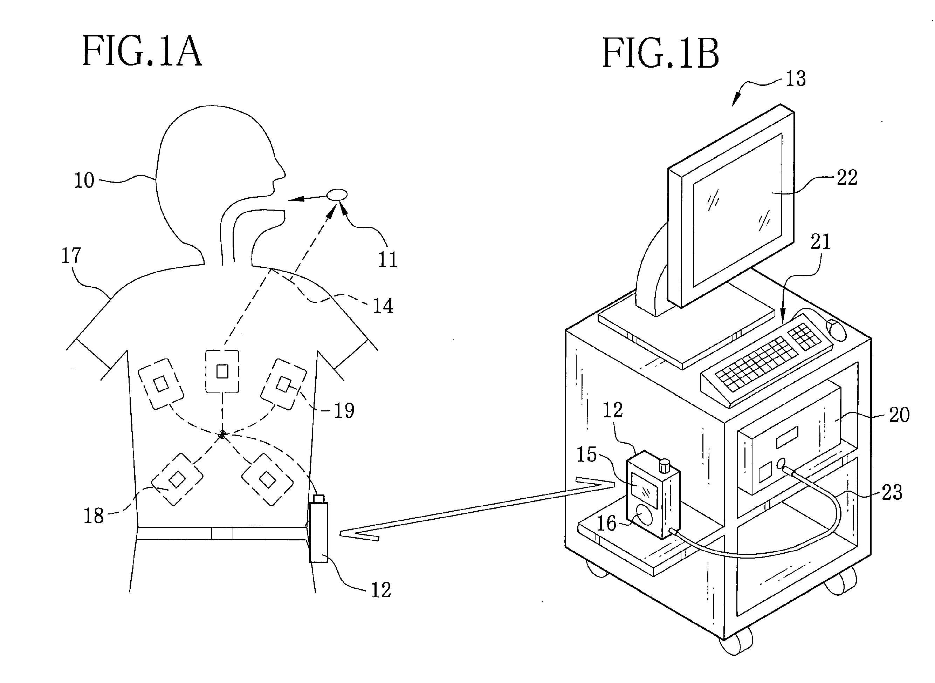

In Figs. 1A and 1B, a capsule endoscope system 2 includes a capsule endoscope 11, a receiver 12, and a workstation (WS) 13. The capsule endoscope 11 is orally swallowed in a human body 10 of a patient. The receiver 12 is attached on a belt with which the receiver 12 is positioned on the human body 10. The workstation 13 retrieves images from the capsule endoscope 11 for a doctor or physician to read the images.

The capsule endoscope 11 picks up an image of an inner surface of the gastrointestinal tract in the human body 10. A radio wave 14 of image data is created and transmitted by the capsule endoscope 11 to the receiver 12. The receiver 12 includes an LCD display panel 15 and an input interface 16. The display panel 15 displays any of various menu regions for settings. The input interface 16 is operable for inputs. The receiver 12 wirelessly receives the radio wave 14 of the image data from the capsule endoscope 11, and stores the image data.

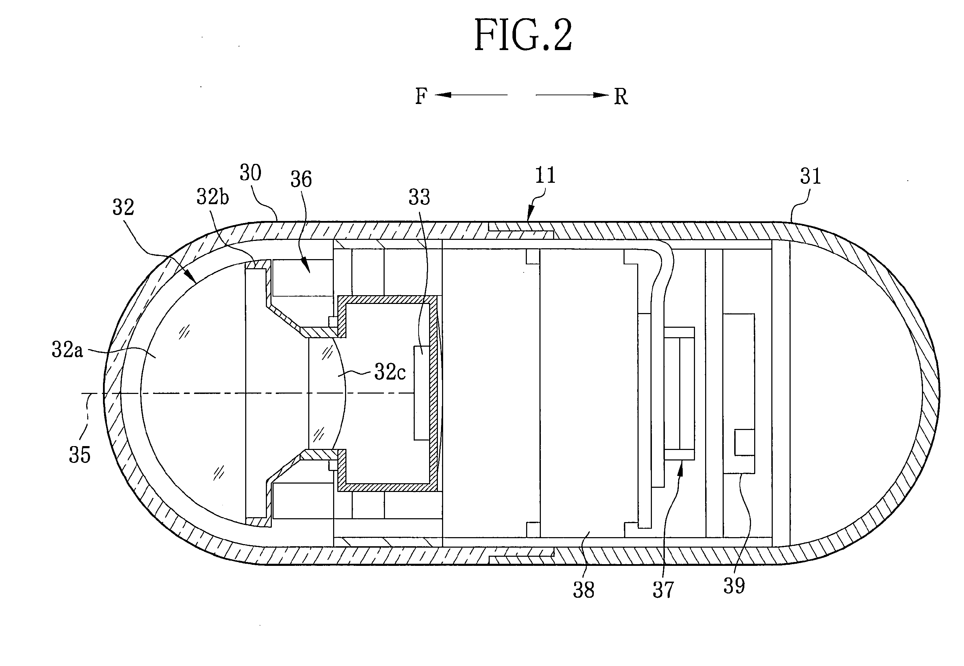

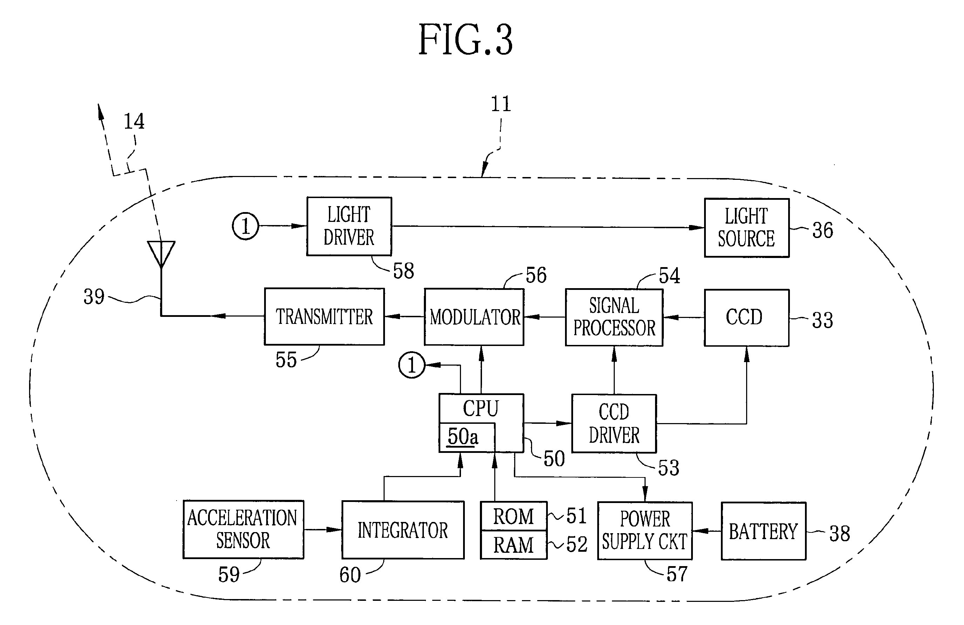

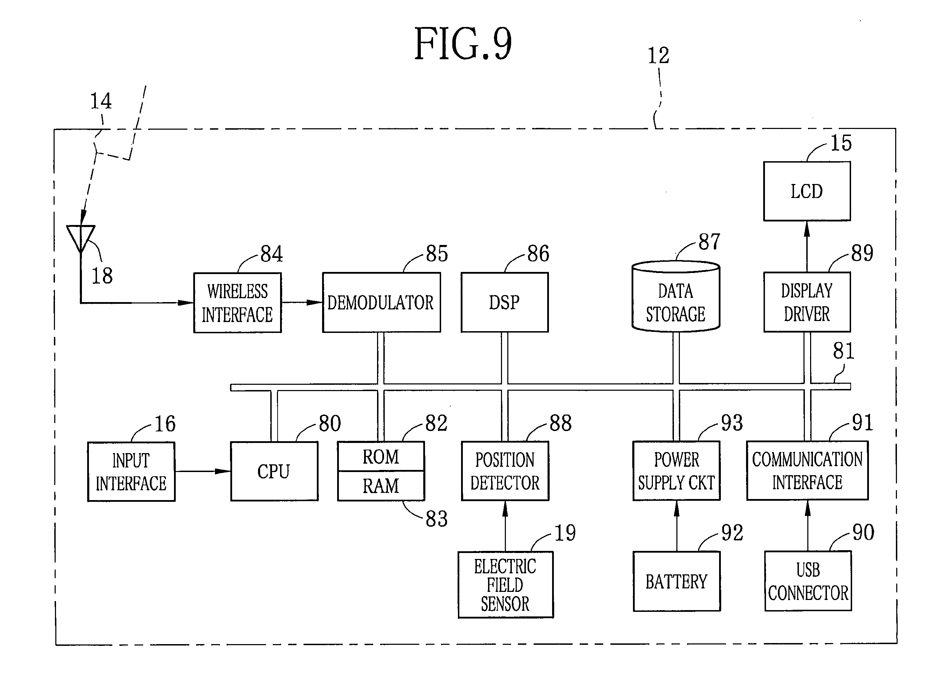

For transmission and reception of the radio wave 14 between the capsule endoscope 11 and the receiver 12, an antenna 39 is disposed in the capsule endoscope 11. See Figs. 2 and 3. Also, a shielding shirt 17 is worn by the human body 10 of the patient. Plural antennas 18 are secured to the inside of the shielding shirt 17. Electric field sensors 19 are incorporated in the antennas 18 for measuring the electric field strength of the radio wave 14 from the capsule endoscope 11. A position detector 88 of Fig. 9 is supplied with result information of the detection from the electric field sensors 19.

The workstation 13 includes a processor 20, an input interface 21 and a monitor display panel 22. The input interface 21 includes a keyboard, mouse and the like. The processor 20 is connected with the receiver 12 by a USB cable 23 or the like, but may be connected by use of infrared communication or other wireless communication. In the receiver 12, data is transmitted to or received from the processor 20. During or after the inspection with the capsule endoscope 11, the processor 20 retrieves image data from the receiver 12, and stores and manages image data for each of numerous patients. Also, the processor 20 creates an image of reproduction from the image data, and causes the monitor display panel 22 to display the image.

In Fig. 2, the capsule endoscope 11 includes a transparent front cover 30 and a rear cover 31 fitted on the transparent front cover 30 in a liquid-tight manner to define an inner space. Each of the front and rear covers 30 and 31 has a cover body of a barrel shape and an end positioned on the cover body in a semispherical shape.

An image pickup assembly 34 is contained in a space defined in the transparent front cover 30 and the rear cover 31, and includes an obj ective optical system 32 and a CCD 33 as image pickup device. The objective optical system 32 receives object light from a body part. The CCD 33 picks up an image by receiving the object light from the body part. An image pickup surface of the CCD 33 is a focal plane in the image pickup assembly 34, and outputs an image signal at numerous pixels when the object light becomes incident upon the image pickup surface.

The objective optical system 32 includes an optical dome 32a, a lens holder 32b and a lens 32c. The optical dome 32a is transparent and disposed about an end of the transparent front cover 30 in a semispherical shape. The lens holder 32b is fitted on a rear end of the optical dome 32a and has a tapered end directed toward the rear. The lens 32c is mounted on the lens holder 32b. An optical axis 35 extends through the center of the objective optical system 32, which has a front view angle of 140-180 degrees and receives image light from an object as an omni-directional image.

In addition to the image pickup assembly 34, various elements are contained inside the front and rear covers 30 and 31, including a light source 36, a circuit board 37, a button type battery 38, and the antenna 39. The light source 36 applies light to a body part of interest. The circuit board 37 includes a wireless transmitter 55 as wireless interface and a power supply circuit 57 of Fig. 3. The antenna 39 emits the radio wave 14 for transmission.

Note that a direction F is defined as a direction of orienting the transparent front cover 30 along the optical axis 35. A direction R is defined as a direction of orienting the rear cover 31 along the optical axis 35. When the capsule endoscope 11 moves in the direction F, the CCD 33 picks up an image of a body part disposed in the front. When the capsule endoscope 11 moves in the direction R, the CCD 33 picks up an image of a body part disposed in the rear.

In Fig. 3, a CPU 50 as pixel number changer controls various elements in the capsule endoscope 11. A ROM 51 and a RAM 52 are connected with the CPU 50. The ROM 51 stores various programs and data for control of the capsule endoscope 11. The CPU 50 reads required programs and data from the ROM 51 by use of the RAM 52, and runs the programs suitably.

A CCD driver 53 and a signal processor 54 are connected with the CCD 33. The CCD driver 53 controls the CCD 33 and the signal processor 54 for imaging at a predetermined frame rate, for example, 2 fps (frames per second). The signal processor 54 processes an image signal from the CCD 33 in processing of correlated double sampling, amplification and A/D conversion, and converts the image signal into image data of a digital form. The converted image data is processed in image processing of gamma correction and the like.

The transmitter 55 is connected with the antenna 39. A modulator 56 is connected between the transmitter 55 and the CPU 50. The modulator 56 modulates digital image data from the signal processor 54 to output the signal of radio wave 14 to the transmitter 55. The transmitter 55 amplifies the signal from the modulator 56, filters the signal by bandpass filtering, and causes the antenna 39 to transmit the radio wave 14.

The power supply circuit 57 is connected with the button type battery 38 and supplies various elements of the capsule endoscope 11 with power. A light driver 58 is controlled by the CPU 50, and drives the light source 36.

An acceleration sensor 59 as a state detector measures an acceleration rate of the capsule endoscope 11 in the direction F/R. An integrator 60 is supplied with information of the measured acceleration rate. When the capsule endoscope 11 moves in the direction F with acceleration or moves in the direction R with deceleration, a measured acceleration rate of the acceleration sensor 59 is defined positive. When the capsule endoscope 11 moves in the direction F with deceleration or moves in the direction R with acceleration, a measured acceleration rate of the acceleration sensor 59 is defined negative. While the capsule endoscope 11 moves in the direction F/R in a manner of uniform motion, or remains stopped in relation to the direction F/R, or moves vertically to the direction F/R, then acceleration measured by the acceleration sensor 59 is zero (0). The integrator 60 integrates the measured acceleration rate from the acceleration sensor 59 for one time at a suitable interval of time, and obtains a moving speed V0 of the capsule endoscope 11 in the direction F/R. The integrator 60 sends data of the moving speed V0 to the CPU 50.

The CPU 50 receives data of the moving speed V0 output successively by the integrator 60, and determines the moving speed V of the capsule endoscope 11 at the given point of time by accumulation of the moving speed V0 from the start of measuring the acceleration rate. When the capsule endoscope 11 moves in the direction F, the moving speed V is positive. When the capsule endoscope 11 remains stopped or moves vertically to the directions F or R, the moving speed V is zero (0). When the capsule endoscope 11 moves in the direction R, the moving speed V is negative.

At each of five times of creating an image, the CPU 50 compares the absolute value |V| of the moving speed with first and second predetermined threshold values TH1 and TH2 (TH1 > TH2). It is possible that the CPU 50 operates for this periodically at one (1) second or so. According to the comparison, the CPU 50 sends a control signal to the CCD driver 53 for changing over the pixel numbers Pmax, Pmid and Plow for images in the CCD 33. The CCD driver 53 drives the CCD 33 according to the control signal from the CPU 50.

If the absolute value |V| of the moving speed is equal to or more than the first threshold value TH1 (|V| ≥ TH1), then the CPU 50 carries out imaging with the high pixel number Pmax which is the maximum assigned in the CCD 33. If the absolute value |V| of the moving speed is less than the first threshold value TH1 and equal to or more than the second threshold value TH2 (TH2 ≤ |V| < TH1), then the CPU 50 carries out imaging with the medium pixel number Pmid which is smaller than the high pixel number Pmax. If the absolute value |V| of the moving speed is less than the second threshold value TH2 (|V| < TH2), or if the absolute value |V| of the moving speed is zero (0) (|V| = 0 or the capsule endoscope 11 is stopped) , then the CPU 50 carries out imaging with the low pixel number Plow which is smaller than the medium pixel number Pmid.

The CPU 50 sends information of the pixel number to the modulator 56. The pixel number received by the modulator 56 is modulated with image data to create the radio wave 14, which is amplified and filtered in bandpass filtering in the transmitter 55, and then transmitted through the antenna 39 wirelessly.

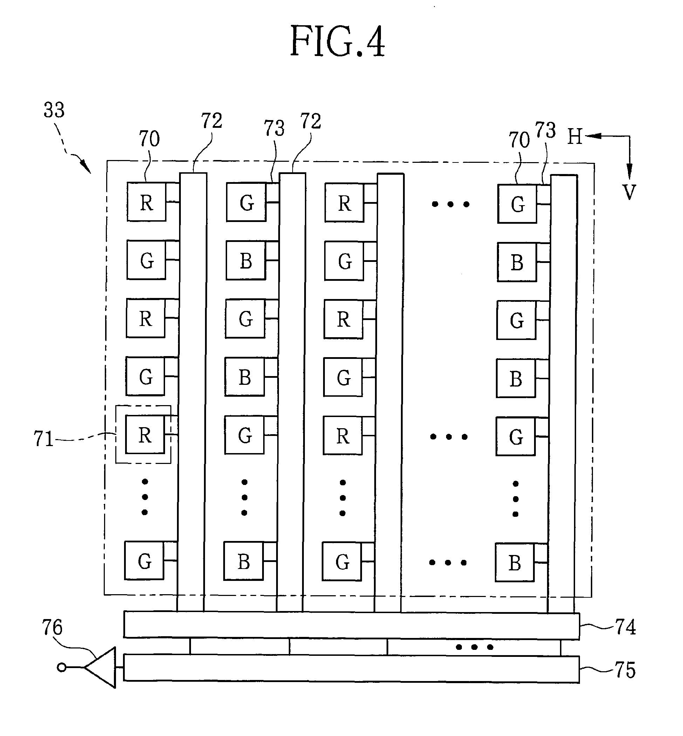

At first, a structure of the CCD 33 is described, prior to description of operation of setting the pixel number. In Fig. 4, the CCD 33 includes plural photodiodes 70 arranged in a two-dimensional manner regularly in the vertical and horizontal directions V and H. An example of the photodiodes 70 is a p-n junction diode as photo diode, and converts incident light into signal charge and stores the signal charge.

There are red, green and blue filters (R, G and B) disposed on the photodiodes 70 on the side of the objective optical system 32. Light of color components corresponding to the color filters is received by the photodiodes 70, so that pixels 71 of the R, G and B colors are defined. The pixels 71 are arranged in the Bayer arrangement known in the art. In short, the green pixels 71 are arranged on lines extending in two diagonal directions. The red pixels 71 are arranged in one line included in two adjacent lines and disposed between the green pixels 71. The blue pixels 71 are arranged in the remaining line included in the two adjacent lines and disposed between the green pixels 71.

A vertical charge transfer path 72 or VCCD is associated with each one line of the photodiodes 70. There is a read gate 73 with which the photodiodes 70 are connected to the vertical charge transfer path 72. In the vertical direction V, The vertical charge transfer path 72 transfers signal charge read from the photodiodes 70 through the read gate 73.

A line memory 74 is connected to each terminal of the vertical charge transfer path 72. A horizontal charge transfer path 75 or HCCD is connected with the line memory 74. The line memory 74 successively receives signal charge of the pixels 71 of one line from the vertical charge transfer path 72, and transfers the signal charge to the horizontal charge transfer path 75 in a selective form, for example, by one line, or for pixels which are included in one line but in one column per two columns. The signal charge read from the line memory 74 is transferred in the horizontal direction H by the horizontal charge transfer path 75.

An output amplifier 76 is connected with a terminal of the horizontal charge transfer path 75. An example of the output amplifier 76 is an FD amplifier (floating diffusion amplifier), which receives signal charge successively from the horizontal charge transfer path 75, and outputs a voltage signal as an image signal by the charge/voltage conversion of the signal charge.

In the present embodiment, a pixel binning process of reading is used as conversion of the pixel number. To transfer signal charge in the vertical charge transfer path 72 or the horizontal charge transfer path 75, signal charge of two or more of the pixels 71 is added up in the vertical charge transfer path 72 or the horizontal charge transfer path 75. A sum as a result of the addition of two or more of the pixels 71 is a signal to represent one pixel. A data size of a final set of image data can be reduced considerably. Apparent sensitivity of the CCD 33 can be raised.

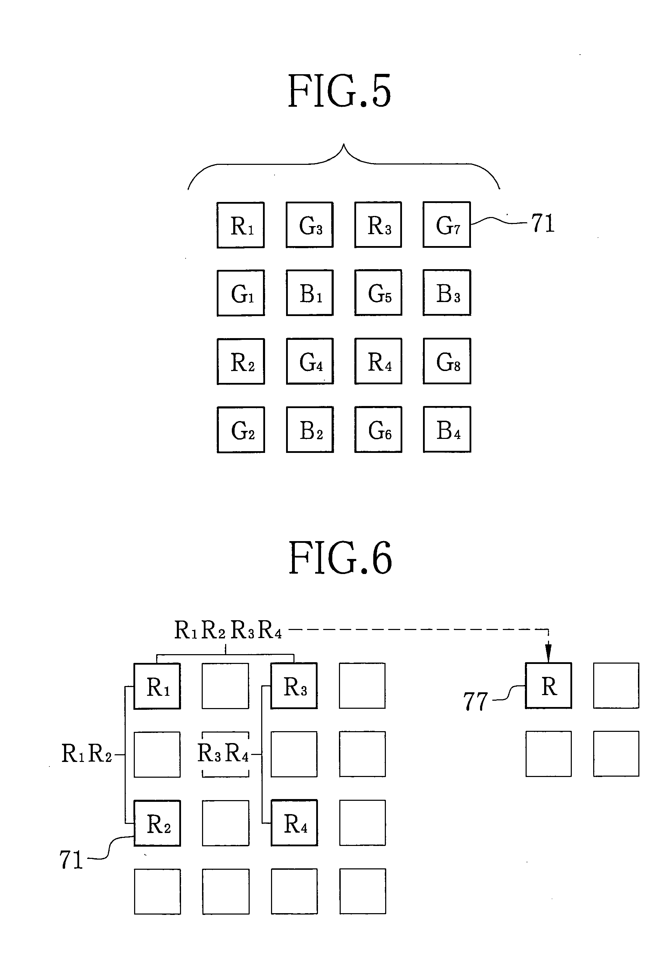

The pixel binning process is schematically illustrated in Figs. 5-8. In Fig. 5, sixteen of the pixels 71 in the matrix form of 4x4 are considered at first, the pixels 71 including 4 red pixels, 4 blue pixels and 8 green pixels. In Fig. 6, the pixel values of the red pixels R1 and R2 of the first line are added up in the vertical charge transfer path 72 to obtain a pixel value R1R2. The pixel values of the red pixels R3 and R4 of the third line are added up in the vertical charge transfer path 72 to obtain a pixel value R3R4. Those two are added up in the horizontal charge transfer path 75 to obtain R1R2R3R4.

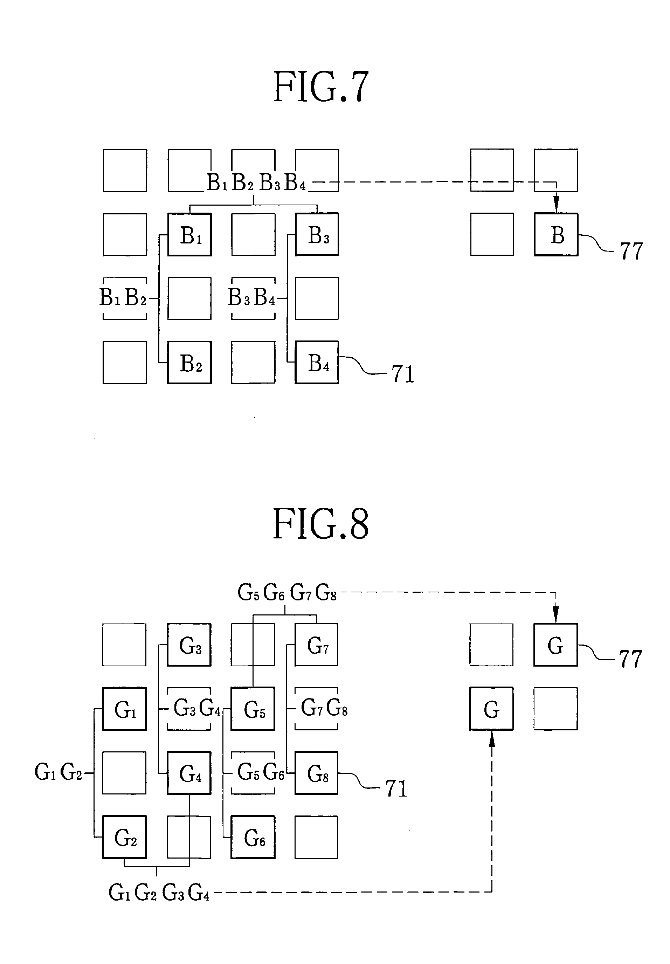

In Fig. 7, similarly, the pixel value of the pixels B1 and B2 of the second line and that of the pixels B3 and B4 of the fourth line are added up by the vertical charge transfer path 72 to obtain the pixel values B1B2 and B3B4. Those are added up by the horizontal charge transfer path 75 to obtain a pixel value B1B2B3B4. In Fig. 8, the pixel values of the pixels G1 and G2 of the first line and those of the pixels G3 and G4 of the second line are added up by the vertical charge transfer path 72 and by the horizontal charge transfer path 75, so that the pixel value G1G2G3G4 is obtained. Also, the pixel values of the pixels G5 and G6 of the third line and those of the pixels G7 and G8 of the fourth line are added up by the vertical charge transfer path 72 and by the horizontal charge transfer path 75, so that the pixel value G5G6G7G8 is obtained.

Then the pixel values R1R2R3R4, B1B2B3B4, G1G2G3G4 and G5G6G7G8 in Figs. 6-8 are rearranged as red, green and blue pixels in combined pixels 77 in a matrix form of 2x2. In normal imaging, signal charge of all the pixels 71 is read discretely to constitute an image at the maximum pixel number Pmax of the CCD 33. Addition and rearrangement of pixels reduce the pixel number to 1/4 as high as the pixel number Pmax. In the present embodiment, the pixel number Pmid for the medium pixel number imaging is set 1/4 as high as the pixel number Pmax. The pixel number Plow for the low pixel number imaging is set 1/16 as high as the pixel number Pmax. Details of pixel binning are disclosed in U.S.P. 6,885,399 (corresponding to JP-A 2000-350099).

In Fig. 9, a CPU 80 controls the entirety of the receiver 12. A ROM 82 and RAM 83 are connected with the CPU 80 by a data bus 81. The ROM 82 stores various programs and data for control of the receiver 12. The CPU 80 reads required programs and data from the ROM 82 by use of the RAM 83, and runs the programs suitably. The CPU 80 is responsive to an input signal from the input interface 16, and operates various elements in the receiver 12.

A receiver interface 84 is connected with the antennas 18. A demodulator 85 is connected with the receiver interface 84. The receiver interface 84 amplifies the radio wave 14 received by the antennas 18, filters the radio wave 14 in the bandpass filtering, and then sends the signal to the demodulator 85. The demodulator 85 demodulates the radio wave 14 from the receiver interface 84 to obtain initial image data and pixel number information. The demodulated image data is output to a DSP (digital signal processor) 86. The pixel number information is stored in data storage 87.

A digital signal processor (DSP) 86 processes the demodulated image data from the demodulator 85 according to signal processing, obtains image data and sends the image data to the data storage 87. An example of the data storage 87 is a flash memory having a storage size as much as 1 Gb. The data storage 87 records and stores image data successively output by the digital signal processor 86 and pixel number information output from the demodulator 85 and combined with the image data.

The position detector 88 detects a present position of the capsule endoscope 11 in the body according to the measured field strength of the radio wave 14 from the electric field sensors 19. The position detector 88 sends position information of the detected present position to the data storage 87. The data storage 87 stores the position information from the position detector 88 with the pixel number information in association with image data from the digital signal processor 86.

Specifically for the position detection of the capsule endoscope 11, for example, data of a data table is experimentally obtained and written in the ROM 82, the data table expressing a relationship between the electric field of the radio wave 14 at the antennas 18 and a position of the capsule endoscope 11 in the body. The ROM 82 is accessed to refer to the data table according to the measured electric field from the electric field sensors 19, for determining the position of the capsule endoscope 11.

Also, a phase difference of the radio wave 14 may be detected for the purpose of position detection, namely a difference in the time of reach of the radio wave 14 to the antennas 18. The phase difference of the radio wave 14 represents relative position or distance between the antennas 18 and the capsule endoscope 11. The position detector 88 operates according to a suitable set of equations for conversion or data tables, and converts the phase difference of the radio wave 14 into a distance between each of the antennas 18 and the capsule endoscope 11 to detect a position of the capsule endoscope 11. Furthermore, a direction of the reach of the radio wave 14 to at least two of the antennas 18 is detected to detect a position of the capsule endoscope 11 according to triangulation on the basis of the base line length defined between the antennas 18.

In addition to the above elements, a display driver 89, a communication interface 91, and a power supply circuit 93 are connected with the data bus 81. The display driver 89 controls the display panel 15 for displaying images. The communication interface 91 transmits data to and receives data from the processor 20 by use of a USB connector 90. The power supply circuit 93 is in contact with a battery 92, and supplies various elements of the receiver 12 with power.

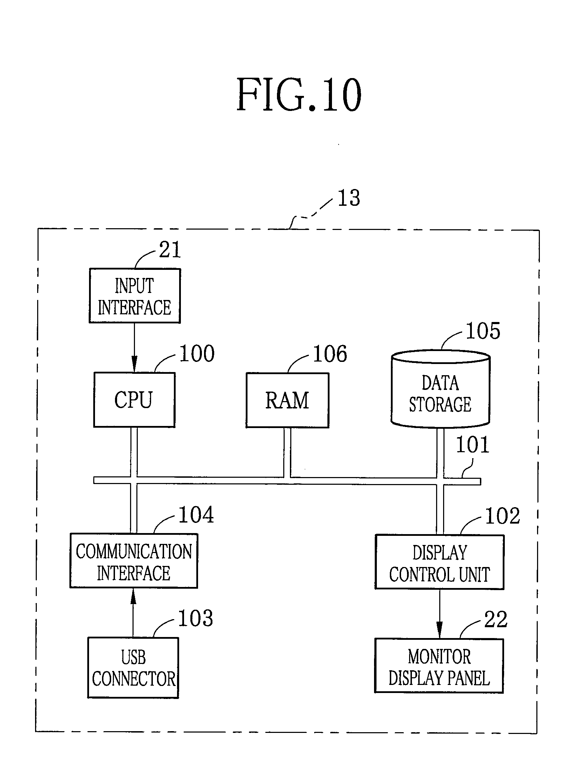

In Fig. 10, a CPU 100 controls the entirety of the workstation 13. The CPU 100 is connected with various elements which include a display driver or display control unit 102, a communication interface 104, data storage 105, and a RAM 106. The display control unit 102 drives and controls the monitor display panel 22 by use of a data bus 101. The communication interface 104 communicates with the receiver 12 through a USB connector 103, and receives image data from the receiver 12. The data storage 105 stores image data from the receiver 12 collectively.

The data storage 105 stores image data, programs and data required for running the workstation 13, and a program for supporting image reading of a doctor or physician. The RAM 106 stores data read from the data storage 105 and intermediate data created arithmetically in the course of calculation.

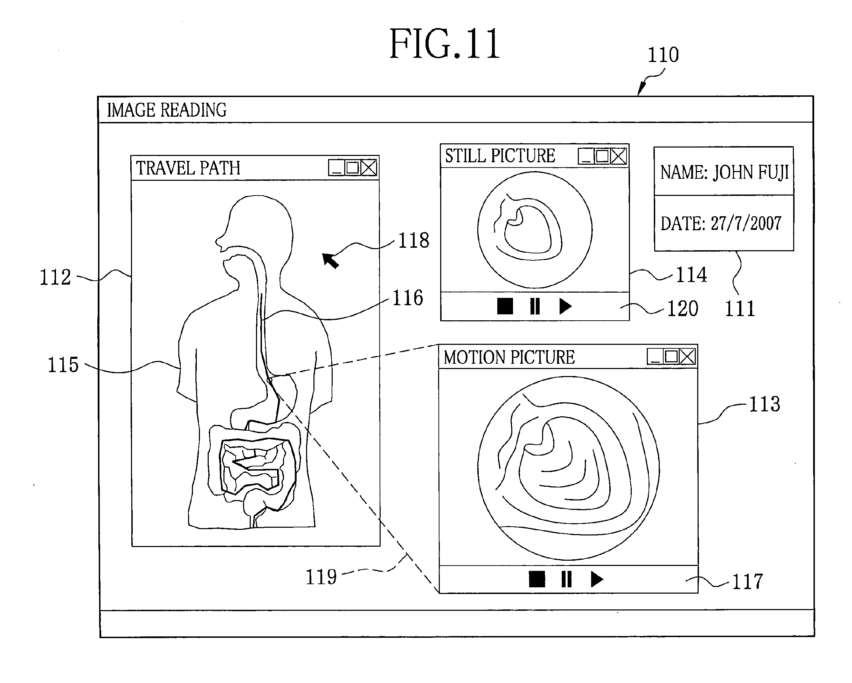

A supporting program is started up, so that a working zone 110 of Fig. 11 is displayed in the monitor display panel 22. A doctor operates the input interface 21 by viewing the working zone 110 to display and edit images or to input information of the inspection. An information area 111, a view area 112, a motion picture area 113 and a still picture area 114 are displayed in the working zone 110. The information area 111 displays various data including patient information of a name and age of a patient with the human body 10, and inspection information such as inspection date.

An anatomical diagram 115 of a simplified form is displayed in the view area 112. A travel path 116 of the capsule endoscope 11 is determined according to position information, and indicated in the anatomical diagram 115. The motion picture area 113 displays images of the pixel number Pmax successively as results of normal imaging among images of image data stored in the data storage 105. Also, images of the pixel numbers Pmid and Plow obtained in the medium and low pixel number imaging are inserted suitably between displayed images of the pixel number Pmax, as results of imaging before and after images of the pixel number Pmax. A control bar 117 is clicked in relation to the motion picture area 113 by use of a cursor 118 for playback, pause and stop. Broken lines 119 extend to connect the motion picture area 113 with an imaged body part on the travel path 116 for the purpose of visually recognizing the imaged body part of the image displayed in the motion picture area 113.

The still picture area 114 displays images of the pixel numbers Pmid and Plow one by one as still images as results of the medium and low pixel number imaging, before and after the image of the pixel number Pmax displayed presently in the motion picture area 113. In a manner similar to the motion picture area 113, a control bar 120 is clicked in relation to the still picture area 114 for playback, pause and stop. Classification of images for the motion and still picture areas 113 and 114 is carried out according to information of pixel numbers. Images of the pixel number Pmax can be also inserted in the still picture area 114. A length of time of displaying images of the pixel number Pmax can be greater than that of displaying images of the pixel numbers Pmid and Plow. Furthermore, it is possible to consider a future structure in which the pixel number Pmax will increase and the pixel number Pmid will become a present value of the pixel number Pmax. To this end, an image of the pixel number Pmid can be displayed in the still picture area 114 by way of a motion image. An image of the pixel number Pmax can be displayed in the motion picture area 113 by way of a still image.

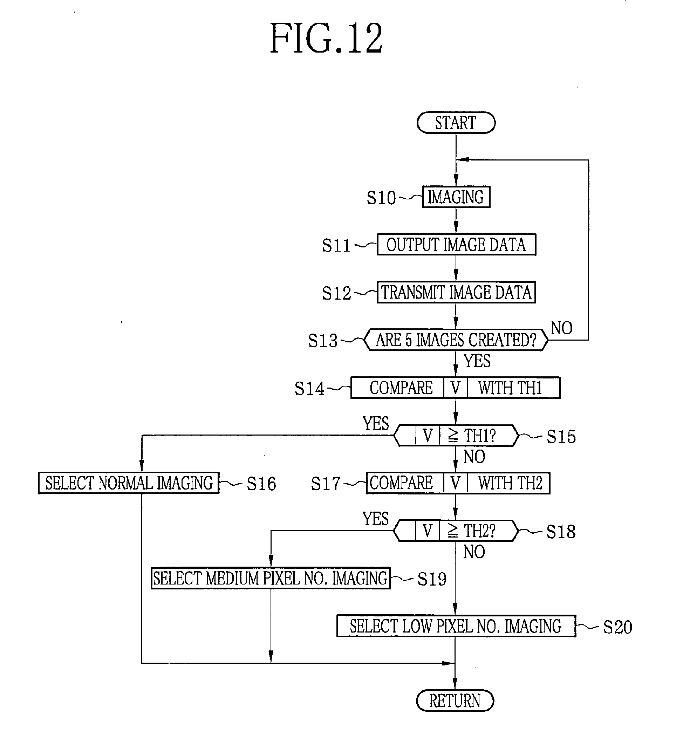

Operation of the capsule endoscope system 2 in the inspection is described now by referring to Fig. 12. At first, a doctor instructs the patient to wear the shielding shirt 17 together with the receiver 12 and the antennas 18. A power source in the capsule endoscope 11 is turned on. The patient is caused to swallow the capsule endoscope 11 orally.

When the capsule endoscope 11 is swallowed in the human body 10, the light source 36 of the capsule endoscope 11 illuminates a body part. The CCD 33 picks up an image of an inner surface of the gastrointestinal tract at a frame rate of 2 fps and pixel number Pmax as a condition of normal imaging. Image light of the body part incident upon the objective optical system 32 is focused on the image pickup surface of the CCD 33, which outputs an image signal.

In the step S11, an image signal output by the CCD 33 is processed by the signal processor 54 in correlated double sampling, amplification and A/D conversion, converted into digital image data and then processed for image processing of various settings. The image data created by the signal processor 54 is sent to the modulator 56.

The modulator 56 modulates image data of a digital form from the signal processor 54 together with pixel number information from the CPU 50, to create the radio wave 14. At the step S12, the radio wave 14 is amplified and filtered for bandpass filtering by the transmitter 55, and transmitted by the antenna 39.

When the antennas 18 in the receiver 12 receive the radio wave 14, the radio wave 14 is amplified and filtered for bandpass filtering by the receiver interface 84, and then demodulated by the demodulator 85 into initial image data and information of the pixel number. The demodulated image data is processed in the digital signal processor 86 for signal processing, and output to the data storage 87 together with the pixel number information.

Also, the electric field sensors 19 measure the field strength of the radio wave 14. A position of the capsule endoscope 11 is detected by the position detector 88 according to a result of measurement of the electric field sensors 19. The position information is obtained by the position detector 88, and is output to the data storage 87. The data storage 87 stores image data, pixel number information and the position information in combination.

Upon swallowing of the capsule endoscope 11 in the human body 10, measurement of an acceleration rate in the acceleration sensor 59 is started. According to a measured result of the acceleration sensor 59, the CPU 50 determines a moving speed V of the capsule endoscope 11.

Five images are taken in the normal imaging, which is expressed by the designation of yes in the step S13. In the step S14, the CPU 50 compares the absolute value |V| of the moving speed with a first threshold value TH1. If the absolute value |V| of the moving speed is equal to or more than the first threshold value TH1 (|V| ≥ TH1 and yes in the step S15), then the CPU 50 sends a control signal to the CCD driver 53 for normal imaging with the pixel number Pmax in the step S16. If the absolute value |V| of the moving speed is less than the first threshold value TH1 (|V| < TH1 and no in the step S15), then the flow proceeds to the step S17.

In the step S17, the CPU 50 compares the absolute value |V| of the moving speed V with a second threshold value TH2. If the absolute value |V| of the moving speed is equal to or more than the second threshold value TH2 (|V| ≥ TH2, and |V| < TH1, and yes in the step S18), then the CPU 50 sends a control signal to the CCD driver 53 for the medium pixel number imaging of the pixel number Pmid which is 1/4 as high as the pixel number Pmax. If the absolute value |V| of the moving speed is less than the second threshold value TH2, or equal to zero (0) (|V| < TH2 or |V| = 0, and no in the step S18), then the CPU 50 sends a control signal to the CCD driver 53 for the low pixel number imaging of the pixel number Plow which is 1/16 as high as the pixel number Pmax.

In response to the control signal from the CPU 50, the CCD 33 is driven by the CCD driver 53 according to a selected one of the normal imaging and medium and low pixel number imaging. If the normal imaging is selected, signal charge of all the pixels 71 is read discretely to create an image of the pixel number Pmax. If the medium or low pixel number imaging is selected, the pixel binning for reading is carried out to create an image of the pixel number Pmid or Plow. When five images are finally created according to any selected mode of imaging, then the CPU 50 compares the absolute value |V| of the moving speed V with the threshold values TH1 and TH2 and determines an imaging condition according to the result of the comparison. This sequence of the operation is continued until the inspection with the capsule endoscope 11 is completed.

The doctor after the inspection connects the receiver 12 with the processor 20 by use of the USB cable 23. Image data from the data storage 87, information of the pixel number associated with the image data, and position information of the image data are uploaded in the data storage 105 of the workstation 13. In the workstation 13, the supporting program is run, with which images are read.

The doctor clicks the control bar 117 for shift according to his or her intention, and reads images of the pixel number Pmax obtained by normal photography and displayed in the motion picture area 113. If a region of interest is found in images in the motion picture area 113 in a form of lesion or the like, he or she terminates indication of the motion picture area 113 in a temporary manner by operating the control bar 117. Images of the pixel number Pmid obtained by the medium pixel number imaging and images of the pixel number Plow obtained by the low pixel number imaging are displayed in the still picture area 114 as still images either before or after the image of the pixel number Pmax present in the motion picture area 113. The doctor reads those images in a detailed manner.

In the present embodiment, an image is formed with the highest pixel number Pmax of the CCD 33 if the moving speed of the capsule endoscope 11 is relatively high in the body (|V| ≥ TH1). An image is formed with the medium pixel number Pmid of the CCD 33 if the moving speed of the capsule endoscope 11 is relatively medium in the body (TH2 < |V| < TH1). An image is formed with the low pixel number Plow of the CCD 33 if the moving speed of the capsule endoscope 11 is relatively low or the capsule endoscope 11 is stopped in the body (|V| < TH2 or |V| = 0). This is effective in reducing the process time and power for use in wirelessly transmitting image data.

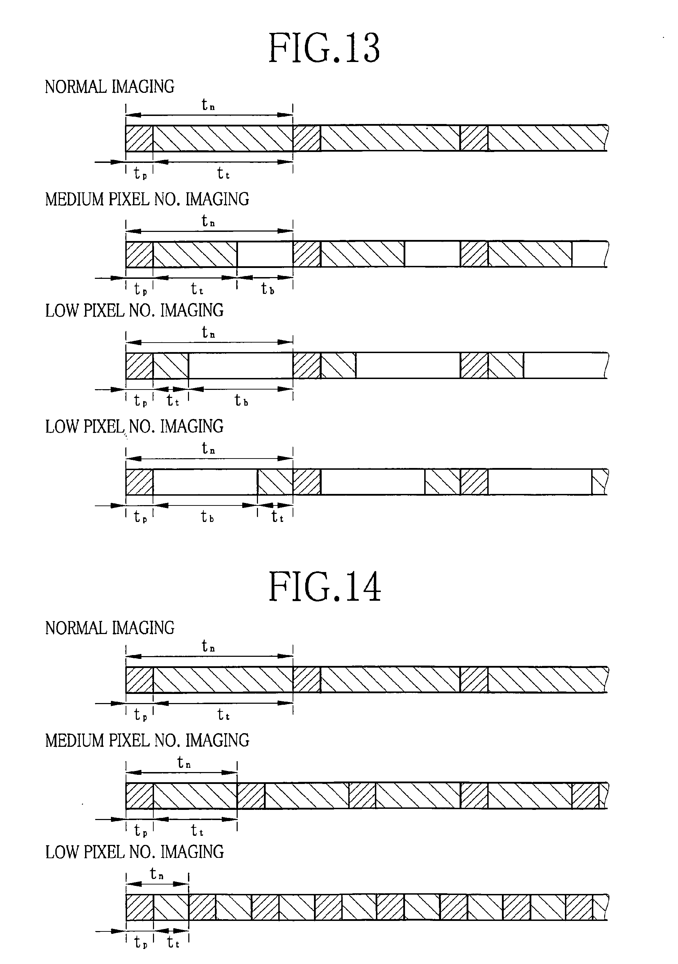

Fig. 13 is referred to for describing the operation specifically. Imaging process time tp, required before an output of digital image data from the signal processor 54 after focusing image light of a body part on to the image pickup surface of the CCD 33, is approximately the same irrespective of the mode of the imaging or the pixel number.

A transmission process time tt, required by modulating image data into the radio wave 14 in the modulator 56 and by transmitting the radio wave 14 with the antenna 39, is longer according to the size of the image data to be transmitted. The transmission process time tt becomes the maximum during the normal imaging in which the pixel number is the highest to maximize the size of image data. At the same time, power required for the transmission becomes the maximum. In the normal imaging, a sum of the imaging process time tp and transmission process time tt is equal to the frame period tn. In the embodiment, tn = 0. 5 second because the frame rate is 2 fps. Also, the embodiment is so constructed that a sum of the imaging process time tp and transmission process time tt is the frame period tn.

In the medium and low pixel number imaging with which sizes of image data are relatively small, a process time tt for transmission is shorter than in the normal imaging, and power for the transmission is relatively small. In the medium and low pixel number imaging, there occurs idle time tb which is included in the frame period tn and in which no imaging or transmission is carried out, under the condition of the constant frame period tn. There are two examples of modes in the medium and low pixel number imaging. In a first one of the examples, the transmission is carried out immediately after imaging, which is indicated in a chart under the chart of the normal imaging. In the second example, a previously obtained image is transmitted before imaging of a succeeding image without transmission immediately after imaging, which is indicated in a lowest chart under the chart of the normal imaging.

The use of the idle time tb is for waiting and stop of various elements in the CCD 33, the CCD driver 53 and the signal processor 54 related to imaging and elements in the transmitter 55 and the modulator 56 related to transmission so as to save power. The capsule endoscope 11 can have a long life as the use of the button type battery 38 can be less quick. Accordingly, the capsule endoscope 11 can continue to operate owing to the long life. There occurs no error in creating images of important body parts, because the button type battery 38 can be prevented from being used up during the imaging.

If the moving speed of the capsule endoscope 11 in the body is relatively high, unusual images can be easily created, because imaged body parts are different between consecutive images. If the moving speed of the capsule endoscope 11 in the body is medium or relatively low, or if the capsule endoscope 11 remains stopped, created images are not always unusual, because a body part is nearly the same or completely the same between consecutive images. Thus, a set of images can be suitable for purpose of image reading in either of precise and quick modes, because importance and rarity of images varies according to the pixel number of imaging in which the images are created.

Also, the capacity of the data storage 87 can be relatively small to reduce the cost for parts, because the size of image data can be smaller than that of image obtained by the imaging of a constant frame rate and constant pixel number.

The total of images treated for the purpose of image reading can be reduced as images of the medium pixel number Pmid and low pixel number Plow are displayed only according to requirement together with images of the pixel number Pmax of normal imaging. Thus, the total of tasks of a doctor or physician can be reduced.

Note that the idle time tb can be eliminated in the manner of the embodiment of Fig. 14. A succeeding image can be created immediately after transmission of image data without waiting time. The frame period tn, namely frame rate, can be dynamically changed according to the mode of the imaging, in addition to the pixel number.

The feature of the structure of Fig. 14 is unsuitable for prolonging the life of the capsule endoscope 11. Then the pixel number is set lower than that for normal imaging to shorten the frame period tn (raise the frame rate) particularly for specific body parts where the moving speed of the capsule endoscope 11 is remarkably high, the specific body parts including an esophagus where passage time of the capsule endoscope 11 is as short as one (1) second, and intestines where the moving speed abruptly rises upon occurrence of peristalsis. For other body parts with moderate moving speed, the structure of the first preferred embodiment is adapted.

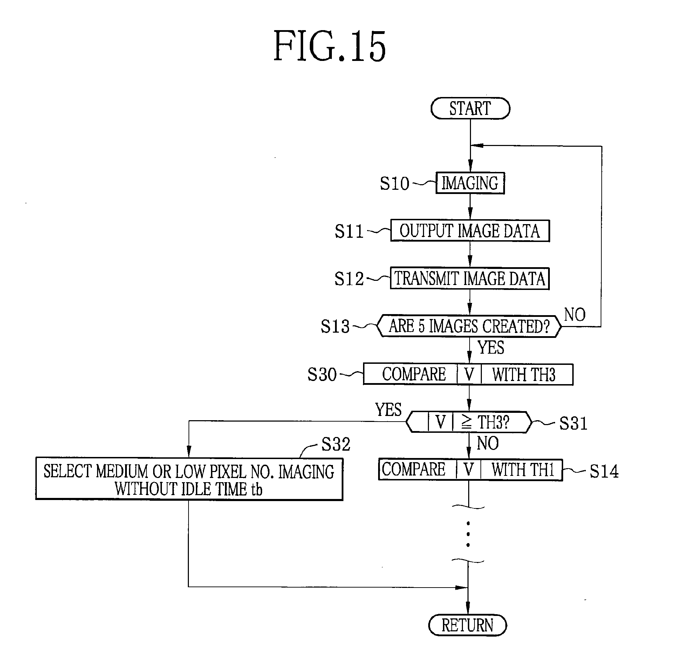

Specifically, the third threshold value TH3 (TH3 > TH1) is predetermined in addition to the threshold values TH1 and TH2. In Fig. 15, image data obtained by imaging is transmitted in the steps S10-S12. Then in the step S30, the absolute value |V| of the moving speed is compared with the third threshold value TH3. If the absolute value |V| of the moving speed is equal to or more than the third threshold value TH3 (|V| > TH3, yes in the step 531), then the imaging is changed over in the step S32 to the medium or low pixel number imaging (without idle time tb) of Fig. 14. If the absolute value |V| of the moving speed is less than the third threshold value TH3 (|V| < TH3, no in the step S31), then the operation proceeds according to the process of the first embodiment. Steps including the step S15 and later are not illustrated.

Passage of the capsule endoscope 11 in the esophagus of the human body 10 immediately after the start of powering the capsule endoscope 11 is estimated. In consideration of this, the medium or low pixel number imaging (without idle time tb) of Fig. 14 can be carried out in a predetermined time from the start of powering the capsule endoscope 11. After the lapse of the predetermined time, the flow can proceed to the process of the first preferred embodiment. It is possible to use a clock 50a of the CPU 50 of Fig. 3 to measure the lapse of the time from the start of powering. Accordingly, it is possible to set a greater number of images by changing the pixel number and the frame period tn specifically for body parts where the moving speed is high. Only the pixel number is changed for the remaining body parts. This is effective in ensuring the imaging by preventing drop of imaging at body parts of the higher moving speed, and in prolonging the useful life of the capsule endoscope 11.

In the present embodiment, a pixel thinning process is preferable as a method of changing the pixel number. Note that the pixel thinning is to form an image by partially retrieving the pixels 71 with selective removal of remaining pixels. The pixel thinning is favorable because of simpler arithmetic operation and shorter process time than the pixel binning. Thus, the pixel thinning is specifically preferable for raising the number of images by raising the frame rate in the present embodiment.

In place of the moving speed of the capsule endoscope 11, a moving distance of the capsule endoscope 11 can be used as capsule endoscope state information to change the pixel number and frame period. The acceleration sensor 59 is a three-axis acceleration sensor for measuring acceleration rates of the axes X, Y and Z, which are the direction F/R, a first direction vertical to the direction F/R, and a second direction vertical to the direction F/R. The measured result of the acceleration sensor 59 is integrated for two times in the integrator 60 at a suitable interval of time, to obtain the moving distance L of the capsule endoscope 11. Data of the moving distance L is input to the CPU 50.

In a manner similar to the first embodiment, the normal imaging is carried out if a shift ΔL as a change in the moving distance L is large per unit time (or at each set of a predetermined number of images), which corresponds to the high moving speed. The medium or low pixel number imaging is carried out if a shift ΔL is small per unit time, which corresponds to the low moving speed. Note that it is also possible to select the modes of imaging according to evaluation of the shift ΔL. For a body part with very large shift ΔL, the medium or low pixel number imaging (no idle time tb) is carried out as illustrated in Fig. 14 to raise the total of images. For other body parts, the normal imaging is carried out, which is in the manner of the first preferred embodiment.

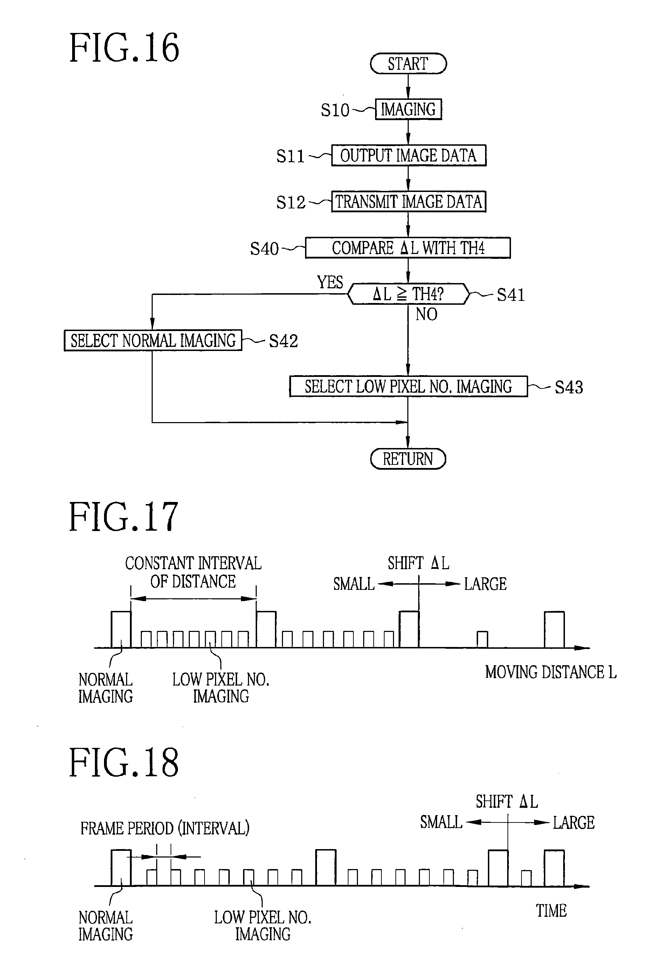

It is also possible in a form like the embodiment of Fig. 16 to take an image normally at each time that the capsule endoscope 11 moves at a predetermined distance and to take an image of a low pixel number between normally taken images.

In Fig. 16, image data obtained by imaging is transmitted in the steps S10-S12 of the third embodiment. In the step S40, the CPU 50 compares the shift ΔL of the capsule endoscope 11 from the point of the previous normal imaging and a predetermined fourth threshold value TH4. If the shift ΔL is equal to or more than the fourth threshold value TH4 (ΔL ≥ H4) in the step S41, then the normal imaging is selected in the step S42 to create an image with the pixel number Pmax. If the shift ΔL is less than the fourth threshold value TH4 (ΔL < TH4) in the step S41, then the low pixel number imaging is selected in the step S43 to create an image with the pixel number Plow and a predetermined frame rate of 2 fps.

Operation of the third preferred embodiment is illustrated in Figs. 17 and 18. In Fig. 17, the moving distance is taken on the horizontal axis. In Fig. 18, the time is taken on the horizontal axis. In Fig. 17, the normal imaging is carried out at a predetermined interval of the distance. The low pixel number imaging is carried out between images of the normal imaging. On the right side with a large shift ΔL, time for moving by a predetermined distance is short, and thus the number of images of the low pixel number imaging is smaller than that on the left side with a small shift ΔL. In Fig. 18, the low pixel number imaging is repeated at a predetermined frame rate or a constant frame period tn between images of normal imaging.

If the capsule endoscope 11 carries out the normal imaging per unit distance of movement, it is likely that a region of interest with a lesion is missed in the imaging, or that the number of images of the region of interest is very small. In contrast, an image is created in the low pixel number imaging during images of the normal imaging. Power for use in the data transmission can be kept small. Also, missing of a region of interest can be prevented. Also, one region of interest possibly appears in plural images. The plural images can be read for the region of interest from the multiple point of view of a doctor.

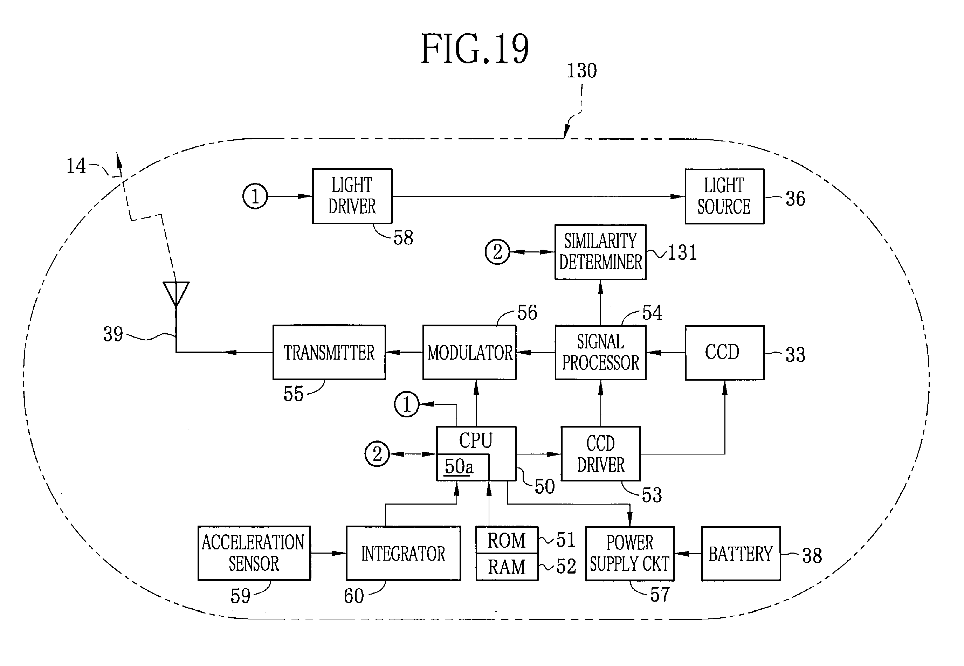

The capsule endoscope state information with which the pixel number is changed is not limited to the moving speed and moving distance of the capsule endoscope 11. Fig. 19 illustrates a preferred embodiment in which the pixel number is changed according to similarity degree of images.

In Fig. 19, a capsule endoscope (CE) 130 includes a similarity determiner 131 for determining similarity degree between two successive images, for example previous image data and present image data. In the similarity determiner 131, there is a frame memory for storing image data of two successive images from the signal processor 54, and for rewriting of image data of an image consecutively at each time that image data is output. The similarity determiner 131 analyzes the form, color and other characteristics of body parts present in the successive images according to the image recognition technically well-known, and obtains the similarity degree SI between the frames according to the analysis. The similarity determiner 131 sends data of similarity degree SI to the CPU 50. If the body part is common between the two images, the similarity degree SI is high. If the body parts are different between the two images, the similarity degree SI is low.

If the similarity degree SI is low, the normal imaging is selected. If the similarity degree SI is high, the medium or low pixel number imaging is selected. This is similar to the first preferred embodiment. Note that it is also possible to select the modes of imaging according to evaluation of the similarity degree SI. For a body part with very low similarity degree SI, the medium or low pixel number imaging (no idle time tb) is carried out as illustrated in Fig. 14 to raise the total of images. For other body parts, the normal imaging is carried out, which is in the manner of the first preferred embodiment.

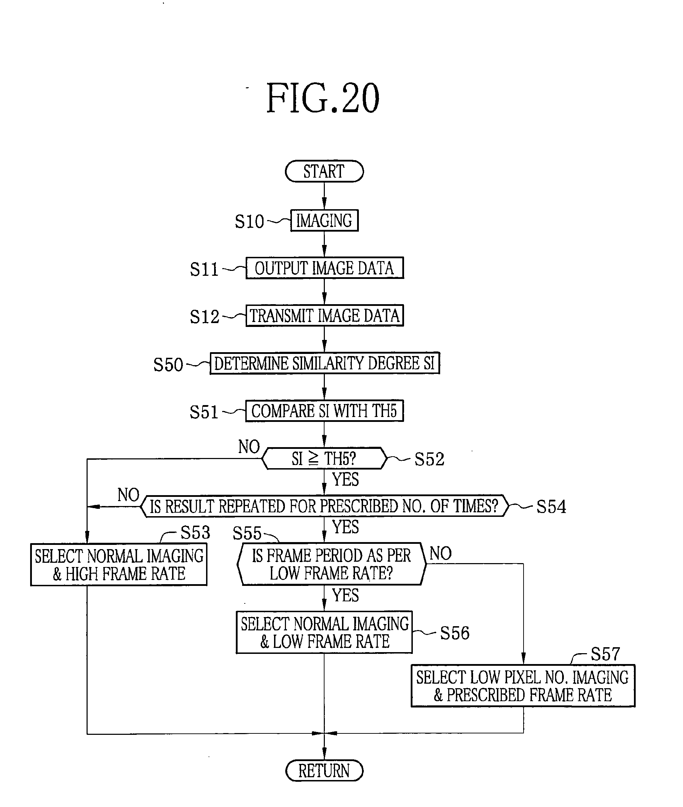

In Fig. 20, one preferred flow of the operation is illustrated. An image is created according to the normal imaging (by way of the third imaging) by lowering the frame rate for an image with higher similarity degree SI among various images. Also, an image is created according to the low pixel number imaging (by way of the fourth imaging) in a period during images of the normal imaging with high similarity degree SI, for the purpose of ensuring imaging of body parts for which the normal imaging is insufficient.

In Fig. 20, image data obtained by imaging is transmitted in the steps S10-S12. In the step S50, the similarity determiner 131 determines information of similarity degree SI of prior and succeeding frames. The CPU 50 receives the information of similarity degree SI from the similarity determiner 131. In the step S51, the information of similarity degree SI is compared with a fifth threshold value TH5 determined previously.

In the step S52, if the similarity degree SI is less than the fifth threshold value TH5 (S < TH5), then the operation proceeds to the step S53. The normal imaging and high frame rate (e.g. 8 fps) are set selectively. In the step S52, if the similarity degree SI is equal to or more than the fifth threshold value TH5 (S ≥ TH5) , then the operation proceeds to the step S54.

In the step S54, if the similarity degree SI is not equal to or more than the fifth threshold value TH5 for a predetermined number of times (e.g. 5 times), then the operation proceeds to the step S53. The normal imaging and high frame rate are set selectively in a manner similar to the situation where the similarity degree SI is less than the fifth threshold value TH5. If the similarity degree SI is equal to or more than the fifth threshold value TH5 for a predetermined number of times (yes in the steps S54 and 55), then the normal imaging and low frame rate (e.g. 1 fps) are set selectively in the step S56. If the frame period is not as long as defined by the low frame rate (no in the step S55), then the low pixel number imaging is set for creating an image at a predetermined frame rate, for example, 4 fps. In other words, in the case of the normal imaging and the low frame rate, a succeeding image of normal imaging is created upon lapse of time of the frame period defined by the low frame rate from a prior image of the normal imaging. The low pixel number imaging is carried out at a predetermined frame rate until lapse of time of the frame period defined by the low frame rate.

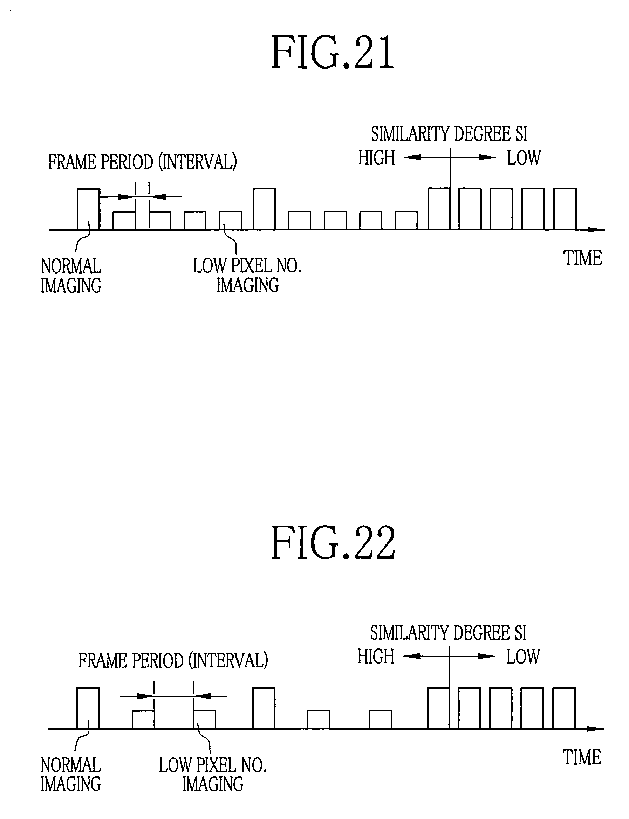

Operation according to the fourth preferred embodiment is illustrated in Fig. 21. The time is taken on the horizontal axis. On the left side with high similarity degree SI, images are created by the normal imaging at a low frame rate. Between the images of the normal imaging, images are created by the low pixel number imaging at a predetermined frame rate, namely at a constant frame period tn. On the right side with low similarity degree SI, an image is created by the normal imaging at a high frame rate.

If the normal imaging at a low frame rate is carried out with high similarity degree SI, it is likely that a region of interest may be missed or only available from a small number of images in a manner similar to the operation in which normal imaging is carried out at each time that the capsule endoscope 11 moves by a predetermined distance. However, in the fourth preferred embodiment, images are recorded in the low pixel number imaging even while the normal imaging is carried out at a low frame rate. Thus, effects similar to those of the third embodiment can be obtained.

Note that a frame rate in the low pixel number imaging selected according to high similarity degree SI is not limited to 4 fps of the above embodiment. For example, the frame rate of the low pixel number imaging may be lower as illustrated in Fig. 22 than that in Fig. 21. As a result, the number of images in the low pixel number imaging is smaller. However, power for use in the imaging can be set lower than that according to Fig. 21.

Also, the frame rate in the low pixel number imaging for a situation of high similarity degree SI may be changed according to the moving speed or moving distance of the capsule endoscope 11. For example, if the moving speed of the capsule endoscope 11 is equal to or near to zero (0) as a state of stop or slow movement, the operation is changed over to that depicted in Fig. 22. If the moving speed of the capsule endoscope 11 is still low but higher than a predetermined low limit, the operation is changed over to that depicted in Fig. 21. It is further possible that the frame rate in the low pixel number imaging for a situation of high similarity degree SI is manually settable by a doctor before swallowing of the capsule endoscope 11 in the human body 10.

Note that the low pixel number imaging according to the third and fourth embodiments is different from that according to the first and second embodiments. In the first and second embodiments, the low pixel number Plow of the low pixel number imaging is 1/16 as high as the pixel number Pmax. In contrast, in the third and fourth embodiments, the low pixel number Plow of the low pixel number imaging can be 1/4 or 1/2 as high as the pixel number Pmax.

Thus, it is possible to combine various modes for ensuring safe recognition of regions of interest without fail and with a reduced power for use, as the pixel number is set according to state information of states of the capsule endoscope 11 in the human body, for example, moving speed, moving distance and similarity degree of images. In the above embodiment, the clock 50a in the CPU 50 is used for changing the frame rate, namely frame period.

In the above embodiments, the steps of the pixel numbers for changeover are two or three. However, the steps of the pixel numbers for use in the present invention may be more than three. The image pickup device in use may be a CMOS image sensor instead of the CCD. Blocks of the CCD driver 53, the signal processor 54 and the like are included in the assembly of the CMOS image sensor. Also, operation time, frame rate and the like may be associated with image data in addition to pixel number information, position information and the like.

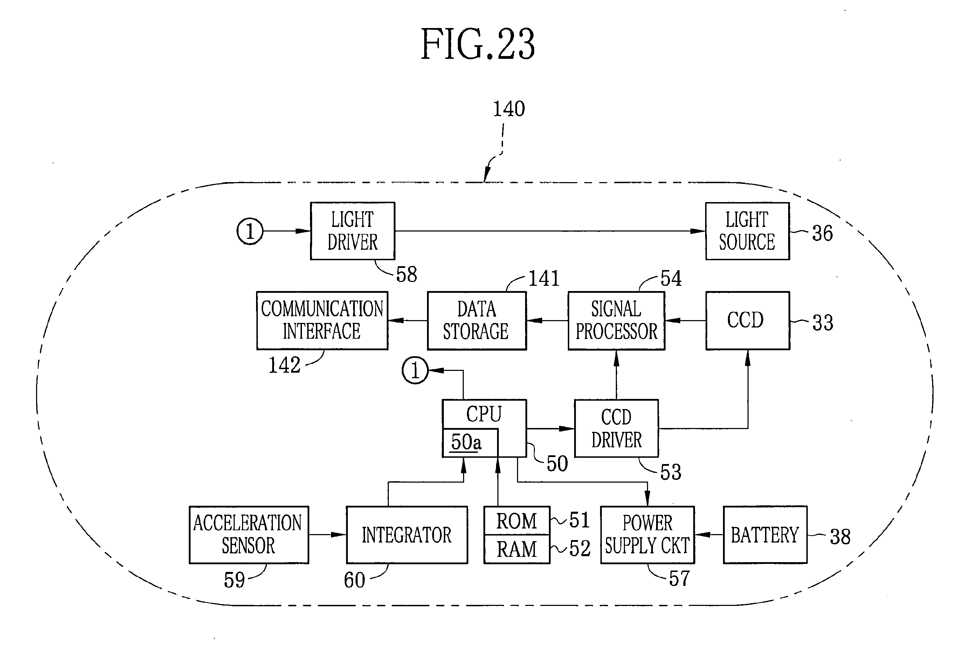

In the above embodiments, image data are transmitted at each time that one image is created. In contrast, image data of plural images may be transmitted after creation of those. In Fig. 23, another preferred capsule endoscope (CE) 140 includes data storage 141 and a communication interface 142. The data storage 141 corresponds to the data storage 87. The communication interface 142 transmits data in the data storage 141 wirelessly or on line as an element in place of the modulator 56. After the capsule endoscope 140 having exited from the body is withdrawn, data in the data storage 141 may be transmitted and input to the processor 20 through the communication interface 142. Effects of the above embodiments can be obtained, because time and power required for transmission can be reduced by reduction in the pixel number.

The threshold values TH1-TH5 for comparison with the moving speed, moving distance, and similarity degree of images may not be fixed but variable, and can be adjusted manually by a doctor or physician. In the above embodiment, the absolute value |V| of the moving speed V is compared with the three threshold values TH1-TH3 at each time of images of a predetermined number or upon lapse of predetermined time, for the purpose of preventing excessively frequent changes in the pixel number. Furthermore, it is possible to change the pixel number at the time of reach to the threshold value ± a tolerable limit in consideration of a hysteresis characteristic in operation of the comparison. The arrangement of the pixels 71 in the CCD 33 and the pixel binning process are not limited to the above embodiments.

In the above embodiment, the electric field sensors 19 are used for obtaining position information. Furthermore, position information may be obtained by use of Hall elements. A magnet is incorporated in the capsule endoscope 11. The Hall elements are associated with the antennas 18, and measure field strength of the magnetic field, so that a position of the capsule endoscope 11 is detected by the position detector 88 according to the measured field strength. Also, an image analysis unit may be incorporated in the receiver 12 for analyzing image data according to the known image recognition technique without use of the electric field sensors 19 or Hall elements. Image data from the capsule endoscope 11 can be analyzed by the image analysis unit to detect a position of the capsule endoscope 11. Images of a specific body part of a typical organ may be prepared as template images. A position of the capsule endoscope 11 may be determined according to degree of coincidence between information of the template images and image from the capsule endoscope 11. In short, any suitable method for detecting a position in a human body, in manners different from the above-described methods, can be used.

Measurement of the moving speed and moving distance is not limited to that according to the above embodiments. For example, position information or operation time is utilized arithmetically to determine the moving speed or moving distance.

In the above embodiment, the pixel binning is used as process of reading. However, other processes may be used, for example, pixel thinning, and pixel averaging of obtaining the average of pixel values between plural pixels including a target pixel and pixels disposed about the target pixel to use the average pixel value as a pixel value of the target pixel.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A capsule endoscope swallowable in a body (10), comprising:
an image pickup device (34) for picking up an image (113, 114) in said body;
a transmitter (55) for externally transmitting information of said image;
a state detector (59) for detecting a capsule endoscope state in said body;
a pixel number changer (50) for changing a pixel number (Pmax, Pmid, Plow) of said image according to information (V, L, ΔL, SI) of said capsule endoscope state.

2. A capsule endoscope as defined in claim 1, wherein said information of said capsule endoscope state is at least one of a capsule moving speed in said body, a capsule moving distance in said body, and similarity degree between plural consecutive images;
said pixel number changer compares said information of said capsule endoscope state with a predetermined threshold value, and determines said pixel number according to a result of said comparison.

3. A capsule endoscope as defined in claim 2, wherein said pixel number changer changes said pixel number stepwise at least between a maximum pixel number of pixels in said image pickup device and a pixel number lower than said maximum pixel number.

4. A capsule endoscope as defined in claim 2, wherein said transmitter transmits said image wirelessly;
when said pixel number is set equal to a maximum pixel number of pixels in said image pickup device, said image pickup device and said transmitter operate for imaging of said image and wireless transmission without idle time;
when said pixel number is set lower than said maximum pixel number, said image pickup device and said transmitter wait during an idle time associated with said imaging of said image and said wireless transmission.

5. A capsule endoscope as defined in claim 2, wherein said transmitter transmits said image wirelessly;
said image pickup device and said transmitter operate for imaging of said image and wireless transmission without idle time irrespective of how said pixel number is set.

6. A capsule endoscope as defined in claim 2, wherein said transmitter transmits said image wirelessly;
when said pixel number is set equal to a maximum pixel number of pixels in said image pickup device, said image pickup device and said transmitter operate for imaging of said image and wireless transmission without idle time;
when said pixel number is set lower than said maximum pixel number, comparison of said information of said capsule endoscope state with said threshold value is considered, for determining a selected one of first and second modes, and in said first mode, said image pickup device and said transmitter wait during an idle time associated with said imaging of said image and said wireless transmission, and in said second mode, said image pickup device and said transmitter operate by shortening said idle time for said imaging of said image at said lower pixel number and said wireless transmission.

7. A capsule endoscope as defined in claim 6, wherein said pixel number changer changes said pixel number by pixel thinning.

8. A capsule endoscope as defined in claim 2, wherein said information of said capsule endoscope state is a capsule moving distance in said body;
said image pickup device creates first images at a regular distance interval according to said capsule moving distance, and creates a second image between said first images;
said pixel number changer sets said pixel number for said first images equal to a maximum pixel number of pixels in said image pickup device, and sets said pixel number for said second image lower than said maximum pixel number.

9. A capsule endoscope as defined in claim 2, wherein said information of said capsule endoscope state is similarity degree between plural consecutive images;
further comprising a frame rate changer for changing a frame rate of said image pickup device according to said similarity degree;
said image pickup device picks up first images at said frame rate set by said frame rate changer, and if said frame rate is made lower than a highest frame rate in said frame rate changer, picks up a second image between said first images;
said pixel number changer sets said pixel number for said first images equal to a maximum pixel number of pixels in said image pickup device, and sets said pixel number for said second image lower than said maximum pixel number.

10. A capsule endoscope as defined in claim 9, wherein said information of said capsule endoscope state further includes at least one of a capsule moving speed in said body and a capsule moving distance in said body;
said frame rate changer sets said frame rate for said second image according to said capsule moving speed or said capsule moving distance.

11. A capsule endoscope as defined in claim 2, wherein said transmitter transmits said information of said pixel number associated with said image.

12. A capsule endoscope as defined in claim 11, wherein said pixel number changer changes said pixel number by at least one of pixel binning, pixel thinning and pixel averaging.

13. A capsule endoscope as defined in claim 2, further comprising data storage for storing images created variously by imaging in said image pickup device;
wherein said transmitter externally transmits said images from said data storage after discharge from said body.

14. A capsule endoscope as defined in claim 2, wherein said pixel number changer sets said pixel number lower according to lowness of said capsule moving speed.

15. A capsule endoscope as defined in claim 2, wherein said pixel number changer sets said pixel number lower according to lowness of said capsule moving distance.

16. A capsule endoscope as defined in claim 2, wherein said pixel number changer sets said pixel number lower according to highness of said similarity degree between plural consecutive images.

17. A capsule endoscope control method for a capsule endoscope swallowable in a body (10), said capsule endoscope including an image pickup device (34), for picking up an image (113, 114) in said body, and a transmitter (55) for externally transmitting information of said image, said capsule endoscope control method comprising steps of:
detecting a capsule endoscope state in said body;
changing a pixel number (Pmax, Pmid, Plow) of said image according to information (V, L, ΔL, SI) of said capsule endoscope state.

18. A capsule endoscope control method as defined in claim 17, wherein said information of said capsule endoscope state is at least one of a capsule moving speed in said body, a capsule moving distance in said body, and similarity degree between plural consecutive images.

19. An information manager for an image (113, 114) from a capsule endoscope swallowable in a body (10), said capsule endoscope including:
an image pickup device (34) for picking up said image in said body;
a state detector (59) for detecting a capsule endoscope state in said body;
a pixel number changer (50) for changing a pixel number (Pmax, Pmid, Plow) of said image according to information (V, L, ΔL, SI) of said capsule endoscope state;
a transmitter (55) for externally transmitting information of said image and said pixel number;
said information manager comprising:
a managing unit (100) for managing said image retrieved from said capsule endoscope, to display said image for image reading; and
a display control unit (100, 102) for changing a displayed form of said image according to said information of said pixel number.

20. An information manager as defined in claim 19, wherein said information of said capsule endoscope state is at least one of a capsule moving speed in said body, a capsule moving distance in said body, and similarity degree between plural consecutive images.

21. An information manager as defined in claim 19, further comprising an input interface operable externally;
wherein said display control unit normally causes displaying an image of a specific pixel number, and when said input interface is operated, causes displaying an image of a pixel number different from said specific pixel number.

22. An information manager as defined in claim 21, wherein said specific pixel number is a maximum pixel number of pixels in said image pickup device.
